(19) **Europäisches Patentamt / European Patent Office / Office européen des brevets**

(11) **EP 4 691 451 A1**

(12) # EUROPEAN PATENT APPLICATION

(43) Date of publication:
**11.02.2026 Bulletin 2026/07**

(21) Application number: **24193463.7**

(22) Date of filing: **08.08.2024**

(51) International Patent Classification (IPC):
**A61K 8/19** (2006.01)    **A61K 8/34** (2006.01)
**A61K 8/73** (2006.01)    **A61Q 11/00** (2006.01)

(52) Cooperative Patent Classification (CPC):
**A61K 8/19; A61K 8/345; A61K 8/73; A61Q 11/00;**
A61K 2800/28

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicant: **Unilever IP Holdings B.V.**
**6708 WH  Wageningen (NL)**

(72) Inventors:
• **DAVIES, Robert Gray**
  **6708 WH Wageningen (NL)**
• **McDonald, Matthew David**
  **6708 WH Wageningen (NL)**
• **OWENS, Jon Gareth**
  **6708 WH Wageningen (NL)**

(74) Representative: **Tansley, Sally Elizabeth**
**Unilever Patent Group**
**Bronland 14**
**6708 WH Wageningen (NL)**

(54) **ORAL CARE COMPOSITION**

(57)    An oral care composition comprising:
i) a calcium carbonate based abrasive,
ii) a polyol selected from glycerol, sorbitol or mixture thereof
iii) gellan.

EP 4 691 451 A1

## Description

### Field of the Invention

The present invention relates to oral care compositions, in particular to oral care compositions with enhanced stain removal and stain prevention.

### Background of the Invention

Abrasives for use in oral care compositions such as dentifrices, are required to be effective in removing extrinsic stains, dental plaque and food debris which builds up on the pellicle on the surface of teeth.

In general, the efficiency of physical removal of stain, plaque and food debris can be increased by using an abrasive having increased abrasivity, or by increasing the level of abrasive incorporated into the composition. However, both these approaches also increase the risk that tooth surfaces may be damaged.

The present invention relates to an oral care composition that effectively removes stains from the teeth.

### Summary of the Invention

The present invention relates to an oral care composition comprising:

    i) a calcium carbonate based abrasive,
    ii) a polyol selected from glycerol, sorbitol or mixture thereof,
    iii) gellan.

The present invention further relates to an oral care composition as described above for use in a method to use in a method to remove stains from the teeth.

### Detailed Description of the Invention

Except in the examples, or where otherwise explicitly indicated, all numbers in this description indicating amounts of material or conditions of reaction, physical properties of materials and/or use may optionally be understood as modified by the word "about".

All amounts are by weight of the composition, unless otherwise specified.

It should be noted that in specifying any ranges of values, any upper value can be associated with any particular lower value.

Where a feature is disclosed with respect to a particular aspect of the invention (for example a composition of the invention), such disclosure is also to be considered to apply to any other aspect of the invention (for example a method of the invention) *mutatis mutandis.*

Any ingredients mentioned in this application that are natural or naturally derived have been sourced from Europe.

The term "oral care composition" refers to a composition that is delivered to the oral surfaces. The composition may be a product which, during the normal course of usage, is not for the purpose of systemic administration or intentionally swallowed but is rather retained in the oral cavity for a time sufficient to contact substantially all of the dental surfaces and/or oral tissues for the purposes of oral activity.

Examples of the oral composition include a toothpaste or a dentifrice, a mouthwash or a mouth rinse, powder (e.g., tooth powder), lozenge, mint, cream, strip or gum (e.g., chewing gum), a film a topical oral gel, and a denture cleanser and the like.

The composition of the invention is used to preferably used to clean the surfaces of the oral cavity.

Accordingly, preferred product forms for compositions of the invention are those which are suitable for brushing and/or rinsing the surfaces of the oral cavity.

The composition of the invention is most preferably in the form of a dentifrice. The term "dentifrice" denotes an oral composition which is used to clean the surfaces of the oral cavity. Such a composition is not intentionally swallowed for purposes of systemic administration of therapeutic agents, but is applied to the oral cavity, used to treat the oral cavity and then expectorated. Typically, such a composition is used in conjunction with a cleaning implement such as a toothbrush, usually by applying it to the bristles of the toothbrush and then brushing the accessible surfaces of the oral cavity.

Preferably the dentifrice/toothpaste is in the form of an extrudable semi-solid such as a cream, paste or gel (or mixture thereof).

A composition according to the invention (such as a dentifrice/toothpaste) will generally contain further ingredients to enhance performance and/or consumer acceptability, in addition to the ingredients specified above.

Compositions of the invention comprise gellan, and preferably further comprise xanthan gum. Preferably the weight ratio of gellan to the xanthan is from 1:5 to 5:1, more preferably from 1:3 to 3:1.

Preferably the level of gellan is from 0.1 to 5 wt% of the total composition, more preferably from 0.3 to 3wt%.

Preferably the level of xanthan, if present. is from 0.1 to 5 wt% of the total composition, more preferably from 0.3 to 3wt%.

Compositions according comprise calcium carbonate particulate abrasive materials usually in amounts between 3 and 60% by weight of the oral care composition.

Precipitated calcium carbonate has a different crystal structure from natural calcium carbonate. It is generally more friable and more porous, thus having lower abrasivity and higher water absorption. For use in the present invention, the particles are small, e.g., having an average particle size of 1-5 microns, and e.g., no more than 0.1 percent, preferably no more than 0.05 percent by weight of particles which would not pass through a 325 mesh. The particles may for example have a D50 of 3-6 microns, for example 3.8-4.9, e.g., about 4.3; a D50 of 1-4 microns, e.g., 2.2-2.6 microns, e.g., about 2.4 microns, and a D10 of 1-2 microns, e.g., 1.2-1.4, e.g., about 1.3 microns. The particles have relatively high water absorption, e.g., at least 25 g/100 g, e.g., 30-70 g/100 g.

In some embodiments, additional calcium-containing abrasives, for example calcium phosphate abrasive, e.g., tricalcium phosphate, hydroxyapatite or dicalcium phosphate dihydrate or calcium pyrophosphate, and/or silica abrasives, sodium metaphosphate, potassium metaphosphate, aluminum silicate, calcined alumina, or other siliceous materials, or combinations thereof are used.

The oral care composition may comprise a hexametaphosphate, a pyrophosphate and mixtures thereof. Preferably the hexametaphosphate is sodium hexametaphosphate. Preferably the pyrophosphate is tetra sodium pyrophosphate, di sodium pyrophosphate or mixtures thereof The total level of hexametaphosphate and a pyrophosphate is preferably from 0.5 to 10 wt% of the total composition more preferably from 1 to 7wt%.

The composition, particularly if a toothpaste preferably comprises an inorganic or a natural or synthetic thickener or gelling agent in proportions of about 0.10 to about 15% by weight depending on the material chosen. These proportions of thickeners in the dentifrice compositions of the present invention form an extrudable, shape-retaining product which can be squeezed from a tube onto a toothbrush and will not fall between the bristles of the brush but rather, will substantially maintain its shape thereon. Suitable thickeners or gelling agents useful in the practice of the present invention include inorganic thickening silicas such as amorphous silicas available from Huber Corporation under the trade designation Zeodent 165, Irish moss, iota-carrageenan, gum tragacanth, and polyvinylpyrrolidone.

Further suitable binders and thickeners can be present and include as sodium carboxymethylcellulose, hydroxyethyl cellulose (Natrosol®), as well as synthetic polymers such as polyacrylates and carboxyvinyl polymers such as Carbopol®.

Compositions according to the invention may comprise a polymeric deposition aid. Preferably the composition comprises acid anhydride polymers, particularly preferred are co-polymers of maleic anhydride with methyl vinylether, in which the anhydride moiety may be in a partially or fully hydrolysed or alcoholysed form. Preferred copolymers include Gantrez(R) polymers such as:

Gantrez S-95: molecular weight 216,000; free acid;
Gantrez S-96: molecular weight 700,000; free acid;
Gantrez S-97: molecular weight 1,500,000; free acid; and
Gantrez MS-955: molecular weight 1,060,000; calcium/sodium salt.

Particularly preferred co-polymers of maleic acid and methyl vinylether have a molecular weight of 1,000,000 or greater and an especially preferred material is Gantrez S-97.

Compositions according to the invention may comprise a tooth whitening agent. The whitening agent preferably comprises a green and/or a blue pigment and/or a green and/or a blue dye. In the context of the present invention a pigment is generally understood to be a shade/material which is insoluble in the relevant medium, at the relevant temperature. This is in contrast to dyes which are soluble. In the context of this invention, the "relevant medium" is human saliva, the liquid medium in which the composition is used, at the temperature of the oral cavity during brushing of the teeth, i.e. up to 37 Degrees C. As a reasonable approximation, the relevant medium may be considered to be water and the relevant temperature to be 25 Degrees C.

Preferably the blue pigment is Pigment Blue 15, more preferably Pigment Blue 15:1, 15:2, 15:3, 15:4, 15:5 or 15:6, most preferably 15:1. A preferred pigment is blue pigment is Phthalocyanine Blue Pigment, CI No. 74160, blue covarine.

The preferred Green pigment is Phthalocyanine Green, preferably Phthalocyanine Green CI-74260.

Preferably the total level of pigment in the composition is from 0.01 wt% to 3 wt, more preferably from 0.02 to 2 wt%.

If the composition is a toothpaste it may be a dual phase paste, with the whitening pigments present in one phase.

Compositions according to the invention may comprise water-soluble or sparingly water-soluble sources of metal salts. Preferred are zinc ions such as zinc chloride, zinc acetate, zinc gluconate, zinc sulphate, zinc fluoride, zinc citrate, zinc lactate, zinc oxide, zinc monoglycerolate, zinc tartrate, zinc pyrophosphate and zinc maleate; also preferred are stannous ions such as stannous fluoride and stannous chloride.

Compositions according to the invention may comprise oral care enzyme systems such as hydrogen peroxide producing enzyme systems (e.g. the oxidoreductase enzyme glucose oxidase), amyloglucosidase, dextranase and/or mutanase, (optionally in the presence of zinc ion providing compounds and/or 8- hydroxyquinoline derivatives), lactoperoxidase, lactoferrin, lysozyme and mixtures thereof.

Compositions of the invention may comprise fluoride sources such as sodium fluoride, stannous fluoride, sodium monofluorophosphate, zinc ammonium fluoride, tin ammonium fluoride, calcium fluoride, cobalt ammonium fluoride and mixtures thereof. Fluoride ion sources may be added to the compositions at a level of about 0.001 wt. percent to about 10 wt. percent, e.g., from about 0.003 wt. percent to about 5 wt. percent, 0.01 wt. percent to about 1 wt., or about 0.05 wt. percent. In some embodiment, the stannous fluoride is present in an amount of 0.1 wt. percent to 2 wt. percent (0.1 wt percent -0.6 wt. percent) of the total composition weight. Fluoride ion sources may be added to the compositions at a level of about 0.001 wt. percent to about 10 wt. percent, e.g., from about 0.003 wt. percent to about 5 wt. percent, 0.01 wt. percent to about 1 wt., or about 0.05 wt. percent. However, it is to be understood that the weights of fluoride salts to provide the appropriate level of fluoride ion will obviously vary based on the weight of the counter ion in the salt, and one of skill in the art may readily determine such amounts. In some embodiment, the fluoride source is a fluoride salt present in an amount of 0.1 wt. percent to 2 wt. percent (0.1 wt percent -0.6 wt. percent) of the total composition weight (e.g., sodium fluoride (e.g., about 0.32 wt. percent).

Some embodiments of the invention are free from fluoride sources, that is the composition comprises less than 0.01 wt% of a fluoride source.

In one embodiment a preferred class of oral care active for inclusion in the compositions of the invention includes agents for the remineralisation of teeth. The term "remineralisation" in the context of the present invention means the *in situ* generation of hydroxyapatite on teeth.

A specific example of a suitable agent for the remineralisation of teeth is a mixture of a calcium source and a phosphate source which, when delivered to the teeth results in the *in situ* generation of hydroxyapatite on teeth.

Illustrative examples of the types of calcium source that may be used in this context (hereinafter termed "remineralising calcium sources") include, for example, calcium phosphate, calcium gluconate, calcium oxide, calcium lactate, calcium glycerophosphate, calcium carbonate, calcium hydroxide, calcium sulphate, calcium carboxymethyl cellulose, calcium alginate, calcium salts of citric acid, calcium silicate and mixtures thereof. Preferably the remineralising calcium source is calcium silicate.

The amount of remineralising calcium source(s) (e.g. calcium silicate) in the composition of the invention typically ranges from 1 to 30%, preferably from 5 to 20% by total weight remineralising calcium source based on the total weight of the oral care composition.

Illustrative examples of the types of phosphate source that may be used in this context (hereinafter termed "remineralising phosphate sources") include, for example, monosodium dihydrogen phosphate, disodium hydrogen phosphate, sodium pyrophosphate, tetrasodium pyrophosphate, sodium tripolyphosphate, sodium hexametaphosphate, potassium dihydrogenphosphate, trisodium phosphate, tripotassium phosphate and mixtures thereof.

Preferably the remineralising phosphate source is a mixture of trisodium phosphate and sodium dihydrogen phosphate.

The amount of remineralising phosphate source(s) (e.g. trisodium phosphate and sodium dihydrogen phosphate) in the composition of this invention typically ranges from 2 to 15%, preferably from 4 to 10% by total weight remineralising phosphate source based on the total weight of the oral care composition.

In some embodiments, the oral care compositions comprise an effective amount of one or more antibacterial agents, for example comprising an antibacterial agent selected from halogenated diphenyl ether (e.g. triclosan), triclosan monophosphate, herbal extracts and essential oils (e.g., rosemary extract, tea extract, magnolia extract, thymol, menthol, eucalyptol, geraniol, carvacrol, citral, hinokitol, magnolol, ursolic acid, ursic acid, catechol, methyl salicylate, epigallocatechin gallate, epigallocatechin, gallic acid, miswak extract, sea-buckthorn extract), bisguanide antiseptics (e.g., chlorhexidine, alexidine or octenidine), quaternary ammonium compounds (e.g., cetylpyridinium chloride (CPC), CPC-claybenzalkonium chloride, tetradecylpyridinium chloride (TPC), N-tetradecyl-4-ethylpyridinium chloride (TDEPC)), phenolic antiseptics, hexetidine furanones, bacteriocins, ethyllauroyl arginate, arginine bicarbonate, a Camellia extract, a flavonoid, a flavan, halogenated diphenyl ether, creatine, sanguinarine, povidone iodine, delmopinol, salifluor, metal ions (e.g., zinc salts, stannous salts, copper salts, iron salts), propolis and oxygenating agents (e.g., hydrogen peroxide, buffered sodium peroxyborate or peroxycarbonate), phthalic acid and its salts, monoperthalic acid and its salts and esters, ascorbyl stearate, oleoyl sarcosine, alkyl sulfate, dioctyl sulfosuccinate, salicylanilide, domiphen bromide, delmopinol, octapinol and other piperidino derivatives, nisin preparations, chlorite salts; parabens such as methylparaben or propylparaben and mixtures of any of the foregoing. One or more additional antibacterial or preservative agents may optionally be present in the composition in a total amount of from about 0.01 wt. percent to about 0.5 wt. percent, optionally about 0.05 wt. percent to about 0.1 wt. percent or about 0.3 percent, by total weight of the composition.

In some embodiments, the oral care compositions also comprise at least one flavorant, useful for example to enhance taste of the composition. Any orally acceptable natural or synthetic flavorant can be used, including without limitation essential oils and various flavoring aldehydes, esters, alcohols, and similar materials, tea flavors, vanillin, sage, marjoram, parsley oil, spearmint oil, cinnamon oil, oil of wintergreen, peppermint oil, clove oil, bay oil, anise oil, eucalyptus oil, citrus oils, fruit oils, sassafras and essences including those derived from lemon, orange, lime, grapefruit, apricot, banana, grape, apple, strawberry, cherry, pineapple, etc., bean-and nut-derived flavors such as coffee, cocoa, cola, peanut, almond, etc., adsorbed and encapsulated flavorants and the like. Also encompassed within flavorants herein are ingredients that provide fragrance and/or other sensory effect in the mouth, including cooling or warming effects. Such ingredients illustratively include menthol, carvone, menthyl acetate, menthyl lactate, camphor, eucalyptus oil, eucalyptol, anethole, eugenol, cassia, oxanone, a-irisone, , thymol, linalool, benzaldehyde, cinnamaldehyde, N-ethyl-p-menthan-3-carboxamine, N, 2, 3-trimethyl-2- isopropylbutanamide, 3- (1-menthoxy) -propane-1, 2-diol, cinnamaldehyde glycerol acetal (CGA), menthone glycerol acetal (MGA) and the like. One or more flavorants are optionally present in a total amount of from about 0.01 wt. percent to about 5 wt. percent, for example, from about 0.03 wt. percent to about 2.5 wt. percent, optionally about 0.05 wt. percent to about 1.5 wt. percent, further optionally about 0.1 wt. percent to about 0.3 wt. percent and in some embodiments in various embodiments from about 0.01 wt. percent to about 1 wt. percent, from about 0.05 to about 2 percent, from about 0.1 percent to about 2.5 percent, and from about 0.1 to about 0.5 percent by total weight of the composition.

In some embodiments, the oral care compositions comprise at least one sweetener, useful for example to enhance taste of the composition. Sweetening agents among those useful herein include dextrose, polydextrose, sucrose, maltose, dextrin, dried invert sugar, mannose, xylose, ribose, fructose, levulose, galactose, corn syrup, partially hydrolyzed starch, hydrogenated starch hydrolysate, ethanol, sorbitol, mannitol, xylitol, maltitol, isomalt, aspartame, neotame, saccharin and salts thereof (e.g. sodium saccharin), sucralose, dipeptide-based intense sweeteners, cyclamates, dihydrochalcones.

One or more sweeteners are optionally present in a total amount depending strongly on the particular sweetener (s) selected, but typically 0.005 wt. percent to 5 wt. percent, by total weight of the composition, optionally 0.005 wt. percent to 0.2 wt. percent, further optionally 0.05 wt. percent to 0.1 wt. percent by total weight of the composition.

In some embodiments, the oral care compositions further comprise an agent that interferes with or prevents bacterial attachment, e.g., ethyl lauroyl arginiate (ELA), solbrol or chitosan, as well as plaque dispersing agents such as enzymes (papain, glucoamylase, etc.).

Mixtures of any of the above described materials may also be used.

The composition according the invention will comprise further ingredients which are common in the art, such as:

antimicrobial agents, e.g. chlorhexidine, sanguinarine extract, metronidazole, quaternary ammonium compounds, such as cetylpyridinium chloride; cetylpyridium chloride clay complex bis-guanides, such as chlorhexidine digluconate, hexetidine, octenidine, alexidine; and

halogenated bisphenolic compounds, such as 2,2' methylenebis-(4-chloro-6-bromophenol);

anti-inflammatory agents such as ibuprofen, flurbiprofen, aspirin, indomethacin etc.;

anti-caries agents such as sodium- and stannous fluoride, aminefluorides, sodium monofluorophosphate, sodium trimeta phosphate and casein;

plaque buffers such as urea, calcium lactate, calcium glycerophosphate and strontium polyacrylates;

vitamins such as Vitamins A, C, D, B (preferably B3) and E;

plant extracts;

plant-derivable antioxidants such as flavonoid, catechin, polyphenol, and tannin compounds and mixtures thereof;

desensitising agents, e.g. potassium citrate, potassium chloride, potassium tartrate, potassium bicarbonate, potassium oxalate, potassium nitrate and strontium salts;

anti-calculus agents, e.g. alkali-metal pyrophosphates, hypophosphite-containing polymers, organic phosphonates and phosphocitrates etc.;

biomolecules, e.g. bacteriocins, antibodies, enzymes, etc.;

flavours, e.g. peppermint and spearmint oils;

proteinaceous materials such as collagen;

preservatives;

opacifying agents;

hyaluronic acid;

amino acids such as arginine;

colouring agents;

pH-adjusting agents;

sweetening agents;

mouth feel agents

pharmaceutically acceptable carriers, e.g. starch, sucrose, water or water/alcohol systems etc.;

surfactants, such as anionic, nonionic, cationic and zwitterionic or amphoteric surfactants; Humectants such as glycerol, sorbitol, propyleneglycol, xylitol, lactitol etc.;

polymeric compounds which can enhance the delivery of active ingredients such as antimicrobial agents can also be included;

buffers and salts to buffer the pH and ionic strength of the oral care composition; and

other optional ingredients that may be included are e.g. bleaching agents such as peroxy compounds e.g. potassium peroxydiphosphate, effervescing systems such as sodium bicarbonate/citric acid systems, colour change systems, and so on.

Examples

The following Examples were prepared.

Table 1

| Material | Example 1 | Example 2 | Example A | Example B |
|---|---|---|---|---|
| FGNC Calcium Carbonate | 40 | 40 | 40 | 40 |
| Sorbitol | 20 | 20 | 20 | 15 |
| Thickening silica | 3.75 | 3.75 | 3.75 | 5.2 |
| Sodium Monofluorophosphate (SMFP) | 1.12 1450ppm | 1.12 1450ppm | 1.12 1450ppm | 1.12 1450ppm |
| SLS | - | - | - | 1.8 |
| High alkaline SLS liquid (30% solution) | 7.58 | 7.58 | 7.58 | |
| Tripotassium Citrate Monohydrate | 0.5 | 0.5 | 0.5 | - |
| Tri sodium phosphate anhydrous | - | - | - | 0.27 |
| Sodium Silicate (36.5%) | 1.56 | 1.56 | 1.56 | - |
| Benzyl Alcohol | 0.5 | 0.5 | 0.5 | 0.5 |
| Cellulose Gum SCMC 9H | | | | 0.55 |
| Gellan | 1 | 0.5 | | |
| Xanthan | | 0.5 | 1 | |
| Water and minors | To 100 | To 100 | To 100 | To 100 |

Stained hydroxyapatite discs (HAP) were used to test the effectiveness of the toothpastes in removing extrinsic tooth stain.

$$\% \text{ stain removal} = \frac{L^* \text{ (cleaned tn) - } L^* \text{ (soiled)}}{L^* \text{ (baseline) - } L^* \text{ (soiled)}} \times 100$$

The average percentage of stain removal for product group was analyzed with a statistical package

| Polymer | % Stain removal (mean $\pm$ SE) |
|---|---|
| Example 1 | 77.29$\pm$3.33 |
| Example 2 | 74.99 $\pm$ 3.07 |
| Example A | 74.99 $\pm$ **3.07** |

(continued)

| Polymer | % Stain removal (mean ± SE) |
|---|---|
| Example B | 61.61 ± 3.26 |

The Examples demonstrate that gellan can enhance the stain removal ability of calcium carbonate.

**Claims**

1. An oral care composition comprising:

   i) a calcium carbonate based abrasive,
   ii) a polyol selected from glycerol, sorbitol or mixture thereof
   iii) gellan.

2. An oral care composition according to claim 1 in which the level of gellan is from 0.1 to 5 wt% of the total composition.

3. An oral car composition according to an preceding clam that further comprises a surfactant.

4. A toothpaste composition according to any preceding claim that further comprises xanthan.

5. An oral care composition according to any preceding claim that is a toothpaste.

6. An oral care composition according to any preceding claim for use in a method to remove stains from the teeth.

Europäisches
Patentamt

European
Patent Office

Office européen
des brevets

# EUROPEAN SEARCH REPORT

Application Number

**EP 24 19 3463**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | JP S62 114905 A (SANEI KAGAKU KOGYO KK) 26 May 1987 (1987-05-26) * example 4 * | 1-3,5 | INV. A61K8/19 A61K8/34 A61K8/73 A61Q11/00 |
| X | DATABASE GNPD [Online] MINTEL; 10 July 2023 (2023-07-10), anonymous: "Midnight Mist Whitening Toothpaste", XP093245894, Database accession no. 10943872 * the whole document * | 1,3-6 | |
| X | CN 103 478 390 B (UNIV HUAQIAO) 15 April 2015 (2015-04-15) * example 3 * * paragraph [0010] * | 1,6 | |
| X | WO 2020/264563 A1 (PROCTER & GAMBLE [US]) 30 December 2020 (2020-12-30) * example 4 * | 1,3-5 | |

TECHNICAL FIELDS
SEARCHED (IPC)

A61K
A61Q

The present search report has been drawn up for all claims

| Place of search | Date of completion of the search | Examiner |
|---|---|---|
| Munich | 10 March 2025 | Hidaoui, Nadia |

EPO FORM 1503 03.82 (P4C01)

**EP 4 691 451 A1**

## ANNEX TO THE EUROPEAN SEARCH REPORT
## ON EUROPEAN PATENT APPLICATION NO.

EP 24 19 3463

10-03-2025

| Patent document cited in search report | | Publication date | Patent family member(s) | | Publication date |
|---|---|---|---|---|---|
| JP S62114905 | A | 26-05-1987 | NONE | | |
| CN 103478390 | B | 15-04-2015 | NONE | | |
| WO 2020264563 | A1 | 30-12-2020 | AU | 2020303738 A1 | 16-12-2021 |
| | | | AU | 2023254930 A1 | 16-11-2023 |
| | | | BR | 112021024290 A2 | 11-01-2022 |
| | | | CA | 3144434 A1 | 30-12-2020 |
| | | | CN | 114040741 A | 11-02-2022 |
| | | | EP | 3990121 A1 | 04-05-2022 |
| | | | JP | 7295279 B2 | 20-06-2023 |
| | | | JP | 2022537782 A | 29-08-2022 |
| | | | US | 2020405593 A1 | 31-12-2020 |
| | | | US | 2020405594 A1 | 31-12-2020 |
| | | | US | 2021378922 A1 | 09-12-2021 |
| | | | US | 2024058231 A1 | 22-02-2024 |
| | | | WO | 2020264563 A1 | 30-12-2020 |